(19) Europäisches Patentamt
European Patent Office
Office européen des brevets

(11) Numéro de publication : **0 303 527 B1**

# (12) FASCICULE DE BREVET EUROPEEN

(45) Date de publication du fascicule du brevet : **30.10.91 Bulletin 91/44**

(51) Int. Cl.[5] : **B01J 29/32**, B01J 29/06, C07C 5/22

(21) Numéro de dépôt : **88401871.4**

(22) Date de dépôt : **20.07.88**

## (54) Catalyseur du type aluminosilicate renfermant au moins un métal noble et son utilisation en isomérisation d'une coupe C8 aromatique.

(30) Priorité : **14.08.87 FR 8711634**

(43) Date de publication de la demande : **15.02.89 Bulletin 89/07**

(45) Mention de la délivrance du brevet : **30.10.91 Bulletin 91/44**

(84) Etats contractants désignés : **BE DE ES GB IT NL**

(56) Documents cités :
**EP-A- 0 031 255**
**EP-A- 0 070 657**
**DE-A- 1 542 559**
**FR-A- 1 510 253**
**US-A- 3 791 963**

(56) Documents cités :
**STUDIES IN SURFACE SCIENCE AND CATALYSIS 28: "New developments in Zeolite science and technology", Proceedings of the 7th International Zeolite Conference, Tokyo, 17-22 août 1986, édité par Y. Murakami et al., pages 121-128, Elsevier, Amsterdam, NL; J.L. GUTH et al.: "New route to pentasil-type zeolites using a non alkaline medium in the presence of fluoride ions"**

(73) Titulaire : **INSTITUT FRANCAIS DU PETROLE**
**4, Avenue de Bois-Préau**
**F-92502 Rueil-Malmaison (FR)**

(72) Inventeur : **Travers, Christine**
**12, Bd du Général de Gaulle**
**F-92500 Rueil Malmaison (FR)**
Inventeur : **Raatz, Francis**
**17, rue Michelet**
**F-92500 Rueil Malmaison (FR)**
Inventeur : **Guth, Jean-Louis**
**59, rue Bellevue Brunstatt**
**F-68200 Mulhouse (FR)**
Inventeur : **Kessler, Henri**
**17, rue de la Forêt**
**F-68270 Wittenheim (FR)**

**EP 0 303 527 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne :

— un catalyseur de type aluminosilicate comprenant une zéolithe de structure MFI, contenant du silicium et de l'aluminium, synthétisée en milieu fluorure et un métal du groupe VIII et,

— l'utilisation de ces catalyseurs dans les réactions d'isomérisation des hydrocarbures C8 aromatiques.

L'art antérieur est notamment illustré par les brevets suivants :

US-A-3.791.963, FR-A-1.510.253, EP-A-70.657, EP-A-31.255, DE-A-1.542.559.

La synthèse dans des milieux fluorures de ce type de zéolithe de structure MFI a déjà été décrite dans le brevet français n° 2567868 et plus récemment dans un article de J.L. GUTH et coll. (Proc. 7th Int. Zeolite Conf, Tokyo, August 1986, p. 121).

Cette synthèse consiste :

a) dans une première étape à former un milieu réactionnel comprenant de l'eau, une source de silice, une source d'alumine, une source d'un agent structurant pouvant fournir des cations organiques choisis dans le groupe constitué par les cations tétrapropylammonium ($TPA^+$) et tétrapropylphosphonium ($TPP^+$), ce milieu réactionnel contenant en outre des anions fluorures. Le pH du milieu est généralement inférieur à 10 et les rapports molaires des divers constituants du milieu réactionnel sont décrits dans le brevet français n° FR2567868,

b) dans une deuxième étape à chauffer le dit milieu réactionnel formé dans l'étape (a) à une température comprise entre 80 et 230°C et de préférence entre 140°C et 210°C, cette seconde étape conduisant à l'obtention d'un solide cristallisé, qui est séparé,

c) dans une troisième étape, à chauffer le solide obtenu à l'issue de l'étape b, à une température supérieure à 400°C de manière à éliminer, par décomposition et éventuellement par combustion si le traitement est réalisé en présence d'oxygène, les espèces organiques fournies par l'agent structurant, et contenues dans le solide après synthèse.

Le pH inférieure à 10 du milieu réactionnel peut être obtenu soit directement à partir de l'un ou plusieurs des produits composant le milieu réactionnel, soit par l'ajout audit milieu d'un acide, d'une base, d'un sel acide, d'un sel basique ou d'un mélange tampon complémentaire.

Les anions fluorures $F^-$ peuvent être introduits dans le milieu réactionnel sous forme de fluorures, comme le fluorure de sodium NaF, le fluorure d'ammonium $NH_4F$, le fluorure acide d'ammonium $NH_4HF_2$, le fluorure de tétrapropylammonium $(C_3H_7)_4NF$, le fluorure de tétrapropylphosphonium $(C_3H_7)_4PF$, ou de composés hydrolysables pouvant libérer des anions fluorures dans l'eau, comme le fluorure de silicium $SiF_4$ ou le fluosilicate de sodium $Na_2SiF_6$.

Le fluorure d'ammonium ou le fluorure d'ammonium acide sont des sels préférés, car ils permettent l'obtention d'une zéolithe de structure MFI facile à transformer en sa forme protonée sans qu'il y ait lieu de procéder à des réactions d'échange d'ions.

De nombreuses sources de silice peuvent être utilisées dans la formation du milieu réactionnel. On peut citer, par exemple :

— les silices sous forme d'hydrogels, d'aérogels, de suspensions colloïdales,

— les silices résultant de la précipitation de solutions de silicates solubles, ou de l'hydrolyse d'esters siliciques comme l'ester tétraéthylique de l'acide monoorthosilicique $Si(OC_2H_5)_4$, ou de complexes comme le fluosilicate de sodium $Na_2SiF_6$ ou d'ammonium $(NH_4)_2SiF_6$,

— les silices préparées par des traitements d'extraction et d'activation de composés cristallisés naturels ou synthétiques, comme les silicates d'aluminium, les aluminosilicates, les argiles, etc...

Les silices utilisées peuvent être divisées ou agglomérées.

Parmi les sources d'alumine utilisables on peut citer les sels d'aluminium (sulfate, nitrate, chlorure, fluorure, acétate par exemple), les hydroxydes et oxydes d'aluminium, les aluminates, les esters comme l'ester tripropylique de l'acide monoorthoaluminique $Al(OC_3H_7)_3$.

Au lieu de partir de sources séparées d'alumine et de silice, on peut aussi utiliser des sources où les deux oxydes sont combinés, comme, par exemple, les gels de silice-alumine amorphes, les aluminosilicates cristallisés, parmi lesquels on peut citer les argiles et les zéolithes.

Les sources de silice et d'alumine peuvent être engagées sous forme soluble ou solide, mais également sous forme d'agglomérats comme des extrudés ou des pastilles. Ce dernier conditionnement convient bien aux sources à base de zéolithes brutes ou modifiées déjà agglomérées, qui peuvent ainsi être transformées, selon le nouveau procédé, en zéolithes préformées.

Les sources d'agent structurant pouvant fournir des cations organiques sont de préférence des cations tétrahydrocarbylammonium, tétrahydrocarbylphosphonium, hydrocarbyl étant avantageusement alkyle et de manière préférée propyle.

Les cations tétrapropylammonium ($TPA^+$) ou tétrapropylphosphonium ($TPP^+$) qui sont les agents structurants préférés sont ajoutés de préférence sous forme de leurs sels, par exemple les bromures, les fluorures, mais ils peuvent également être engendrés in situ à partir de tripropylamine ou de tripropylphosphine et d'un halogénure de propyle.

Les acides ou sels acides, les bases ou sels basiques ajoutés éventuellement en complément pour amener le pH du milieu réactionnel à la valeur désirée peuvent être choisis parmi les acides courants, comme l'acide fluorhydrique HF, l'acide chlorhydrique HCl, l'acide nitrique $HNO_3$, l'acide sulfurique $H_2SO_4$, l'acide acétique $CH_3COOH$, ou les sels acides comme le fluorure acide d'ammonium $NH_4HF_2$, le fluorure acide de potassium $KHF_2$, le sulfate acide de sodium $NaHSO_4$, le sulfate acide de potassium $KHSO_4$, le phosphate acide de sodium $NaH_2PO_4$ et les bases courantes comme l'ammoniaque $NH_4OH$, la soude NaOH, la potasse KOH ou les sels basiques courants comme les carbonates acide $NaHCO_3$ ou neutre $Na_2CO_3$ de sodium, l'acétate de sodium $CH_3COONa$, les sulfures neutres $Na_2S$ ou acide NaHS de sodium ou les mélanges tampons comme acide acétique $CH_3COOH$-acétate de sodium $CH_3COONa$, ammoniaque $NH_4OH$-chlorure d'ammonium $NH_4Cl$.

La morphologie, la taille et la cinétique de formation de cristaux de zéolithes obtenues selon le procédé de l'invention peuvent être modifiées par l'introduction dans le milieu réactionnel de sels complémentaires comme le chlorure de sodium NaCl, le chlorure de potassium KCl, le chlorure d'ammonium $NH_4Cl$, le sulfate de sodium $Na_2SO_4$ et/ou de cristaux (broyés ou non) de composés solides apparentés aux zéolithes préparées par le procédé de l'invention.

TABLEAU I : Caractéristiques du spectre de diffraction X des zéolithes de structure MFI selon l'invention.

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08 - 11,26 | FF | 4,06 - 4,10 | ff | 2,772 - 2,793 | ff |
| 9,94 - 10,20 | mf | 3,99 - 4,05 | f | 2,725 - 2,749 | ff |
| 9,68 - 9,90 | f | 3,83 - 3,89 | F | 2,677 - 2,697 | ff |
| 8,98 - 9,08 | ff | 3,80 - 3,86 | m | 2,648 - 2,670 | ff |
| 8,00 - 8,09 | ff | 3,74 - 3,78 | mf | 2,605 - 2,619 | ff |
| 7,40 - 7,52 | ff | 3,70 - 3,74 | mf | 2,581 - 2,597 | ff |
| 7,03 - 7,22 | ff | 3,63 - 3,67 | mf | 2,545 - 2,557 | ff |
| 6,64 - 6,84 | f | 3,58 - 3,62 | ff | 2,508 - 2,526 | ff |
| 6,30 - 6,42 | f | 3,46 - 3,50 | ff | 2,479 - 2,501 | ff |
| 5,95 - 6,07 | f | 3,42 - 3,46 | f | 2,407 - 2,419 | ff |
| 5,67 - 5,79 | f | 3,38 - 3,42 | ff | 2,393 - 2,401 | ff |
| 5,54 - 5,61 | f | 3,33 - 3,37 | f | 2,326 - 2,340 | ff |
| 5,32 - 5,42 | ff | 3,29 - 3,33 | ff | 2,314 - 2,332 | ff |
| 5,10 - 5,23 | ff | 3,23 - 3,27 | ff | 2,195 - 2,209 | ff |
| 5,01 - 5,08 | f | 3,16 - 3,20 | ff | 2,104 - 2,120 | ff |
| 4,95 - 5,03 | f | 3,12 - 3,16 | ff | 2,077 - 2,095 | ff |
| 4,84 - 4,93 | ff | 3,08 - 3,12 | ff | 2,070 - 2,084 | ff |
| 4,59 - 4,64 | ff | 3,03 - 3,07 | f | 2,004 - 2,022 | f |
| 4,44 - 4,50 | ff | 2,976 - 3,020 | f | 1,985 - 2,005 | f |
| 4,34 - 4,40 | f | 2,943 - 2,962 | f | 1,944 - 1,964 | ff |
| 4,23 - 4,29 | f | 2,855 - 2,881 | ff | 1,907 - 1,922 | ff |
| | | | | 1,866 - 1,881 | ff |

FF = très fort ; F = fort ; mF = moyen à fort ; m = moyen
mf = moyen à faible ; f = faible ; ff = très faible

Les solides obtenus par la procédure de synthèse décrite précédemment sont des zéolithes de structure MFI dont les diagrammes de diffraction des rayons X ont des caractéristiques correspondant aux spécifications du tableau 1. Ces zéolithes de structure MFI ont comme formule chimique approchée après calcination exprimée sous forme oxyde :

$$M_{2/n}O, Al_2O_3, xSiO_2$$

où x peut varier de 12 à 1000 et où M représente le ou les cations de compensation de valence n. Le point important est que ces solides contiennent, après l'étape de synthèse et également après l'étape d'élimination des composés organiques, de l'élément fluor. La teneur en fluor dans la zéolithe déterminée par analyse élémentaire est comprise pour les solides calcinés, c'est-à-dire ceux résultant de l'etape (c) décrite précédemment, entre 0,02 et 1,5% en poids, avantageusement entre 0,1 et 1,0% et de préférence entre 0,2 et 0,8%.

La présence de fluor dans les zéolithes de structure MFI préparées selon l'invention confère à ces solides des propriétés, notamment des propriétés acides et d'échange ionique, tout à fait différentes de celles des zéolithes de structure MFI synthétisées de manière classique, c'est-à-dire en milieu alcalin (US 3.702.886 par exemple). Après synthèse et élimination des composés organiques par calcination (étapes a, b, c), les solides selon l'invention sont caractérisés par un spectre de vibration infrarouge qui présente, comme l'illustre la figure pour des teneurs en fluor de 0,8% (courbe 1), de 0,2% (courbe 2) et de 0,05% (courbe 3) des bandes attribuées classiquement aux groupes Si-OH (zone 3730-3750 $cm^{-1}$) et aux groupes Al-OH structuraux (région 3580-3640 $cm^{-1}$) très peu intenses par rapport à celles d'une zéolithe de structure MFI classique, de même rapport Si/Al égal à 22 (courbe 4, % F = 0).

L'absence ou la quasi absence de groupes Al-OH structuraux dans les zéolithes selon l'invention est confirmée par les capacités d'échange ionique de ces solides. En effet les capacités d'échange ionique pour des cations tels que par exemple $Na^+$, $K^+$, $Ga^{3+}$, $Pt(NH_3)_4^{2+}$ etc sont très inférieures aux capacités d'échange théoriques totales que l'on peut calculer à partir de la teneur en aluminium de la charpente cristalline.

Ces solides ne présentant pas ou peu d'hydroxyles structuraux et dont la capacité d'échange est très réduite possèdent de manière surprenante des propriétés acides remarquables. Ainsi la thermodésorption d'ammoniac qui permet de rendre compte de l'acidité globale d'un solide (nombre et force des différents types de sites acides) montre que les solides comprenant du fluor incorporé dans la structure sont très acides. Les spectres de thermodésorption d'ammoniac sont comparables à ceux que l'on obtiendrait avec des zéolithes de structure MFI classiques, cependant l'acidité des solides selon l'invention est d'une nature différente.

Sans nous lier à une théorie particulière, on peut proposer par exemple, que ces solides ont, à la place d'une partie au moins

des sites 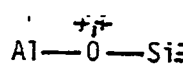 classiques,

des sites du type :

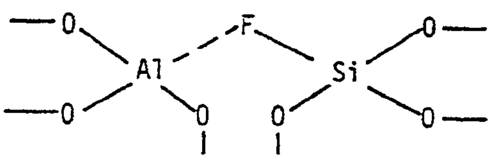

La nature précise des sites acides présents dans les solides selon l'invention reste à préciser, cependant il est clair que ces sites sont liés en grande partie à la présence de fluor et se distinguent de par leur nature des sites acides des zéolithes MFI classiques.

L'introduction de fluor dans les zéolithes est une méthode qui a déjà été proposée pour augmenter l'acidité de ces solides (S. KOWALAK, React. Kinet. Catal. Lett, 27, 1985 p. 441 et J. Chem. Soc. Farad. Trans 1, 82, (1986), 2151 ; J. MIALE and C. CHANG US 4.540.841). Cependant dans l'art antérieur le fluor est introduit dans la zéolithe par des modifications réalisées après la synthèse. En d'autres termes on réalise une synthèse classique, c'est-à-dire en milieu alcalin puis on traite le solide par une technique permettant en principe de fixer du fluor. Ces techniques précédemment proposées, souffrent généralement de gros défauts. Elles sont par exemple, comme c'est le cas quand on traite le solide par du fluor gazeux, susceptibles de conduire à une dégradation de l'ordre cristallin (US 4.297.335). Dans la présente préparation du catalyseur, le fluor est introduit dans la zéolithe au niveau de la synthèse et permet au contraire d'aboutir à des solides très bien cristallisés.

Par des traitements particuliers il est possible d'éliminer partiellement ou totalement le fluor contenu dans les solides entrant dans la composition des catalyseurs selon l'invention sans altérer leur cristallinité. Une technique que l'on peut utiliser pour défluorer les solides consiste à procéder à un traitement dans une solution d'ammoniaque à des températures comprises par exemple entre l'ambiante et 200°C (traitement en autoclave sous pression autogène). L'élimination partielle ou complète du fluor conduit :

— d'une part à l'apparition dans le spectre IR de deux bandes situées vers 3740 et 3608 $cm^{-1}$ correspondant, d'après les attributions admises dans la littérature scientifique, aux groupes silanols terminaux et aux groupes Al-OH structuraux respectivement et

— d'autre part à la restauration de la capacité d'échange ionique telle qu'on peut la calculer à partir de la teneur en aluminium de charpente des solides.

Ainsi en fonction du traitement de défluoration, on peut obtenir pour un même rapport Si/Al de charpente, des solides contenant une quantité de groupes Al-OH et Si-OH ainsi qu'une capacité d'échange ionique variables. Un solide partiellement défluoré contient donc outre des sites acides classiques du type Al-OH pouvant jouer le rôle de sites d'échange, des sites acides particuliers dont la nature exacte n'est pas encore totalement élucidée mais qui résultent indéniablement de l'introduction dans les solides de fluor lors de la synthèse.

C'est cette particularité des solides que l'on a mise à profit pour préparer des catalyseurs bifonctionnels contenant au moins un métal du groupe VIII choisi parmi le platine et le palladium et susceptibles par exemple d'isomériser un hydrocarbure $C_8$ aromatique et plus généralement une coupe $C_8$ aromatique et dont les propriétés acides sont d'un type nouveau.

La présente invention concerne donc un catalyseur du type aluminosilicate caractérisé par la composition suivante exprimée en poids :

a) 0,01 à 1,5% d'au moins un métal choisi parmi le platine et le palladium,

b) 0 à 99,49% d'une matrice choisie dans le groupe formé par l'alumine, la silice, la magnésie, une argile et toute combinaison d'au moins deux des composés précités et

c) 0,50 à 99,99% d'une zéolithe synthétisée en milieu fluorure ayant un rapport molaire $SiO_2/Al_2O_3$ compris entre 12 et 1000, la zéolithe ayant une teneur en fluor comprise entre 0,02 et 1,5% en poids, le fluor étant incorporé lors de la synthèse, la dite zéolithe étant aussi caractérisée par un diagramme de diffraction X présenté dans le tableau I.

La zéolithe a en général la formule chimique approchée suivante :

$$M_{2/n}O, Al_2O_3, xSiO_2,$$

où

M représente un proton et/ou un cation métallique,

n est la valence du cation,

x est un nombre comprise entre 12 et 1000.

Après synthèse en milieu fluorure, le solide peut être soumis si besoin est, à un traitement de défluoration permettant d'ajuster sa capacité d'échange ionique, à la teneur en métal du groupe VIII que l'on veut déposer. Plus la teneur en fluor sera faible, plus la teneur en platine ou palladium pourra être élevée.

Le traitement de défluoration est plus ou moins sévère suivant le niveau de défluoration désiré. Il consiste en un ou plusieurs traitements successifs du solide, à reflux dans une solution d'ammoniaque de normalité comprise entre 0,05 et 5 N et de préférence entre 0,1 et 3 N, pendant une durée comprise entre 0,5 et 5 heures et de préférence entre 1 et 4 heures avec un rapport v/p défini comme le volume de solution par rapport au poids de solide sec compris entre 5 et 50 $cm^3g^{-1}$ et de préférence entre 10 et 30 $cm^3g^{-1}$. Le solide, après chaque lavage est ensuite abondamment lavé à l'eau distillée et séché à l'étuve. Après ces traitements et suivant leur sévérité, la teneur en fluor du solide est comprise entre 0,9 et 0,01% en poids. Si on élimine, par des traitements répétés, sensiblement la totalité du fluor, on aboutit encore à des solides qui se distinguent en particulier par leur spectre IR dans la région 3800-3500 $cm^{-1}$ des zéolithes de structure MFI classiques de même rapport Si/Al de charpente : les solides contenus dans le catalyseur selon l'invention possèdent une proportion plus importante de groupes Si-OH.

Le solide partiellement ou totalement défluoré peut être soumis tel quel au dépôt du métal du groupe VIII ou mis en forme par toutes les techniques connues de l'homme du métier. Par métal du groupe VIII, on entend le platine et le palladium. Il peut en particulier être mélangé à une matrice généralement amorphe, par exemple, à une poudre humide de gel d'alumine. Le mélange est ensuite mis en forme, par exemple par extrusion au travers d'une filière. La teneur en zéolithe du support ainsi obtenu est généralement comprise entre 0,5 et 99,99% et avantageusement comprise entre 40 et 90% poids. Elle est plus particulièrement comprise entre 60 et 85% en poids par rapport à l'ensemble zéolithe et matrice.

La teneur en matrice du catalyseur est avantageusement comprise entre 10 et 60% et de préférence entre 15 et 40% en poids. La mise en forme peut être réalisée avec d'autres matrices que l'alumine, telles que, par exemple la magnésie, la silice-alumine, les argiles naturelles (kaolin, bentonite), et par d'autres techniques que l'extrusion telles que le pastillage ou la dragéification. Le métal hydrogénant du groupe VIII, de préférence Pt ou Pd est ensuite déposé sur le support par tout procédé connu de l'homme du métier et permettant le dépôt du métal sur la zéolithe. On peut utiliser la technique d'échange cationique avec compétition où l'agent compétiteur est de préférence le nitrate d'ammonium, le rapport de compétition étant au moins égal à 50, et avantageusement 50 à 150. Dans le cas du platine ou du palladium, on utilise soit un complexe tétrammine du platine, soit un complexe tétrammine du palladium ; ces derniers se déposeront alors pratiquement en totalité sur la zéolithe. Cette technique d'échange cationique peut également être utilisée pour déposer directement le Platine sur la poudre de zéolithe, avant son mélange éventuel avec une matrice. Le dépôt du métal est suivi d'une calcination sous air ou $O_2$ durant 0,5 à 10 heures entre 300 et 600°C et de préférence entre 350 et 500°C durant 1 à 4 heures. On procède ensuite à une réduction sous hydrogène à une température comprise entre 300 et 600°C pendant 1 à 10 heures. De préférence, on opèrera entre 350 et 550°C pendant 2 à 5 heures. La teneur en métal du groupe VIII (Pt ou Pd) déposée sur le catalyseur et obtenue à l'issue de l'échange dépend du taux de défluoration du solide ; elle se situe entre 0,01 et 1,5% en poids par rapport à l'ensemble du catalyseur et de préférence entre 0,03 et 0,4% en poids.

Afin de déposer, quelle que soit la teneur en fluor, la quantité de platine ou de palladium désirée, on peut déposer ce dernier, non plus directement sur la zéolithe, mais sur le liant aluminique, avant ou après l'étape de mise en forme en mettant en oeuvre un échange anionique avec de l'acide hexachloroplatinique, de l'acide hexachloropalladique ou du chlorure de palladium en présence d'un agent compétiteur, l'acide chlorhydrique. On peut ainsi déposer dans tous les cas jusqu'à 0,5% de platine et de préférence entre 0,03 et 0,4% poids. Après dépôt de platine, le catalyseur est comme précédemment soumis à une calcination entre 300 et 600°C puis réduit sous hydrogène comme indiqué dans le cas précédent. Cette dernière procédure permet d'utiliser la zéolithe sous sa forme totalement fluorée et de déposer une quantité de métal supérieure à la capacité d'échange de la zéolithe, qui peut atteindre 1,5% poids du catalyseur.

Le catalyseur bifonctionnel obtenu par les procédures précédentes est mis en oeuvre par exemple, dans les réactions d'isomérisation d'une coupe C8 aromatique comprenant soit uniquement un mélange de xylènes, soit un mélange de xylènes et de l'éthylbenzène. L'isomérisation des alkyls aromatiques, et en particulier des xylènes, revêt une importance commerciale considérable. Ce sont essentiellement l'ortho et le paraxylène qui sont les produits les plus recherchés, et en particulier le paraxylène qui est utilisé comme intermédiaire dans la fabrication des fibres polyester, la principale utilisation de l'orthoxylène étant la préparation de l'anhydride phtalique. Le méta-xylène présentant un moindre intérêt industriel, il est intéressant de le transformer par isomérisation en ortho et paraxylène. L'éthylbenzène étant difficilement séparable par distillation (les points d'ébullition des différents composés sont très proches) du mélange des xylènes, se trouve très souvent dans la charge d'isomérisation des C8 aromatiques.

Les exemples qui suivent précisent l'invention sans toutefois en limiter la portée, ils sont donnés pour une charge constituée uniquement de métaxylène mais sont facilement transposables à une charge plus complexe comprenant essentiellement du métaxylène et de l'éthylbenzène.

Dans le cas d'une charge comprenant de l'éthylbenzène, le mécanisme de la transformation est un mécanisme bifonctionnel acide qui passe par la formation d'intermédiaires déshydrogénés nécessitant la présence d'un métal du groupe VIII comme fonction hydrogénante/déshydrogénante. Dans le cas d'une charge comprenant uniquement des xylènes, le mécanisme d'isomérisation est un mécanisme essentiellement monofonctionnel acide, mais la présence d'un métal hydrogénant est indispensable pour la stabilité et même la sélectivité du catalyseur.

EXEMPLE 1 : Préparation des zéolithes A et B entrant dans la composition du catalyseur selon l'invention.

On prépare deux zéolithes de structure MFI de rapports atomiques Si/Al proches de 20 et 250 à partir d'une même source d'aluminium et de silicium, à savoir du Tixolex 28 partiellement désaluminé et en utilisant deux rapports atomiques F/Si différents dans les deux mélanges réactionnels.

Le Tixolex 28 est un aluminosilicate de sodium commercialisé par Rhône Poulenc et caractérisé par les rapports atomiques Si/Al = 7,3 et Na/Al = 1,1. On prépare la forme partiellement désaluminée de la manière suivante : 60 g de Tixolex 28 sont agités pendant 3 heures à température ambiante avec 600 ml de $HNO_3$ M/2. Le produit obtenu est filtré et lavé à l'eau jusqu'à pH 7. Après séchage à 80°C, il est conservé dans une humidité relative de 80%. La composition pondérale est la suivante 76,10% $SiO_2$ ; 5,46% $Al_2O_3$ ; 0,24% $Na_2O$ ; 17,63% $H_2O$ totale.

On a prépare deux mélanges réactionnels A, B dont les compositions molaires et pondérales sont indiquées dans le tableau 2. Pour cela, on ajoute sous agitation le mélange de $NH_4F$, $N(C_3H_7)_4{}^+ Br^-$ et eau au Tixolex partiellement désaluminé. La cristallisation des deux mélanges réactionnels A, B est réalisée dans deux autoclaves, dont les revêtements internes sont constitués de polytétrafluoroéthane, à 190°C pendant 3,5 jours.

TABLEAU 2

| | | Tixolex partiellement désaluminé $SiO_2$ | Tixolex partiellement désaluminé $Al_2O_3$ | $NH_4F$ | $N(C_3H_7)_4$ Br | $H_2O$ |
|---|---|---|---|---|---|---|
| A | moles | 0,2 | 0,0084 | 0,04 | 0,1 | 1,6 |
| A | g | 15,8 | | 1,48 | 26,6 | 28,8 |
| B | moles | 0,2 | 0,0084 | 0,25 | 0,1 | 1,6 |
| B | g | 15,8 | | 9,25 | 26,6 | 28,8 |

Après cristallisation les solides sont filtrés et lavés avec une solution de diéthylamine à 10% puis avec une solution d'eau chaude. Les solides sont ensuite séchés à 80°C. L'analyse cristallographique montre que les produits A, B sont bien des zéolithes de structure MFI dont le diagramme de diffraction des rayons X correspond aux spécifications du tableau 1. L'analyse chimique des produits A et B après calcination sous air à 550°C est la suivante :

| Produits | A | B |
|---|---|---|
| (Si/Al) atomique | 22 | 240 |
| F (% poids) | 0,8 | 0,5 |

EXEMPLE 2 : Catalyseur B1 conforme à l'invention

Le solide B de l'exemple 1 est mis en forme par extrusion avec un liant de type aluminique à raison de 80% en poids de zéolithe et 20% en poids de liant. Le métal du groupe VIII, ici le platine est déposé sur les extrudés par échange ionique avec compétition. Le sel de platine utilisé est le complexe de Keller $Pt(NH_3)_4Cl_2$ et l'agent compétiteur $NH_4NO_3$. Le rapport de compétition $NH_4{}^+/2\ Pt(NH_3)_4{}^{2+}$ est égal à environ 50. Le dépôt de platine est suivi d'une calcination sous air à 500°C pendant deux heures. Le solide est réduit ensuite sous hydrogène in situ à 450°C pendant deux heures. Le catalyseur ainsi obtenu est référencé B1.

Le solide B de départ ayant une teneur en fluor importante (0,5% en poids), la teneur en platine atteinte par échange est assez faible. Le catalyseur B1 a une teneur en platine de 0,06% en poids. Le spectre de diffraction des rayons X est sensiblement comparable à celui de l'exemple 1. Le catalyseur B1 est testé en isomérisation du méta-xylène à une température de 400°C, une pression de 15 bar, un rapport $H_2$/métaxylène (molaire) de 4 et une vitesse spatiale (pph) de 3,6. Les performances catalytiques sont reportées dans le tableau 3.

Elles sont définies par :

$$\text{Conversion du m-xylène (\%)} = \frac{\text{Masse de m-xylène dans la charge-masse de m-xylène dans la recette}}{\text{masse de m-xylène dans la charge}} \times 100$$

$$\text{Sélectivité en isomérisation} = \frac{\text{Masse de o-xylène + masse p-xylène}}{\text{(masse des produits)}} \times 100$$

$$\text{Approche à l'équilibre sur le p-xylène} = \frac{\text{nombre de moles de p-xylène dans la recette}}{\text{nombre de moles de p xylène à l'équilibre thermo-dynamique.}}$$

$$\text{Rendement en isomérisation} = \frac{\text{Conversion x sélectivité}}{100}$$

$$\text{Rendement en C8 aromatique} = \frac{\text{(masse des C8 aromatiques) dans la recette}}{\text{Masse totale des C8 aromatiques dans la charge.}} \times 100$$

EXEMPLE 3 : Catalyseur B2 conforme à l'invention.

Le solide B de l'exemple 1 est soumis à une étape de défluoration qui consiste à porter le solide à reflux pendant 4 h dans une solution d'ammoniaque 0,2 N, avec un rapport volume de solution par rapport au poids de solide sec de 20 $cm^3g^{-1}$. Le solide est ensuite lavé abondamment à l'eau distillée puis remis en suspension pour effectuer un second échange. Après filtration et lavage, le solide est séché à l'étuve à 120°C. Sa teneur en fluor est alors de 0,10%. Le solide est alors mis en forme par extrusion dans les mêmes conditions que le catalyseur B1 et du platine est déposé par échange compétitif comme décrit ci-dessus dans l'exemple 2. Cependant la capacité d'échange après défluoration étant multipliée par un facteur 4 environ, on ajuste la quantité de complexe de Keller à introduire afin d'obtenir une teneur en platine équivalente à celle du catalyseur B1 (0,06%). Après dépôt du platine le catalyseur est calciné sous air et réduit sous hydrogène dans les mêmes conditions que celles de l'exemple 2. Le catalyseur obtenu est référencé B2.

Les performances de ce catalyseur testé en isomérisation selon les conditions de l'exemple 2 figurent dans le tableau 3. Elles sont inférieures en sélectivité en isomérisation et en rendement en C8 aromatiques à celles du catalyseur B1 contenant une teneur en fluor plus élevée et une teneur en métal identique.

EXEMPLE 4 : Catalyseur B3 conforme à l'invention.

Le catalyseur B3 diffère du catalyseur B2 en ce qu'on essaie d'atteindre une défluoration sensiblement totale. Pour ce faire on procède à une étape de défluoration plus sévère que celle décrite dans l'exemple 3. On effectue 4 traitements successifs dans les conditions suivantes : le solide est porté à reflux pendant 4 h dans une solution d'ammoniaque 1,5 N, avec un rapport volume de solution par rapport au poids de zéolithe sec de 20 $cm^3g^{-1}$. Entre chaque traitement, le solide est abondamment lavé à l'eau distillée puis remis en suspension. Après le dernier lavage, le solide est séché à l'étuve à 120°C. Sa teneur en fluor est alors de 0,02%. Après défluoration, le solide est mis en forme dans les mêmes conditions que celle de l'exemple 2. La zéolithe ayant retrouvé pratiquement toute sa capacité d'échange (calculée par rapport à la teneur en Al de charpente), on ajuste la quantité de platine à introduire afin d'obtenir une teneur en Pt sur le catalyseur final équivalente à celle des catalyseurs B1 et B2. Le dépôt de platine est effectué selon la méthode décrite dans l'exemple 2. Après dépôt du platine, le catalyseur est calciné sous air et réduit sous hydrogène dans les mêmes conditions que celles de l'exemple 2. Le catalyseur ainsi obtenu est référencé B3, ses performances catalytiques en isomérisation selon les conditions de l'exemple 2 sont données dans le tableau 3.

EXEMPLE 5 : Catalyseur C de comparaison.

Le catalyseur C est une zéolithe de structure MFI synthétisée en milieu basique conventionnel dont la description figure dans le brevet US 3.702.886. Cette zéolithe est synthétisée avec un rapport Si/Al de 240 et ne contient pas du tout de fluor après synthèse. Le dépôt de platine est effectué selon la méthode décrite dans l'exemple 2. La teneur en platine est équivalente à celle des catalyseurs B1, B2, B3. Ce catalyseur est mis en forme et activé dans les mêmes conditions que celles décrites dans l'exemple 2. Les performances du catalyseur C testé en isomérisation selon les conditions de l'exemple 2 figurent dans le tableau 3. On constate que si ces performances sont inférieures à celles du catalyseur B1 conforme à l'invention, elles sont néanmoins assez proches de celles du catalyseur B3, c'est-à-dire du catalyseur conforme à l'invention ne contenant pratiquement plus de fluor.

EXEMPLE 6 : Catalyseur Al conforme à l'invention.

On prépare un catalyseur dans les mêmes conditions que celles décrites dans l'exemple 2 mais en utilisant la zéolithe A de l'exemple 1 dont le rapport atomique Si/Al est égal à 22. Le catalyseur ainsi obtenu est référencé Al et est testé dans les mêmes conditions que les catalyseurs précédents. Les performances catalytiques du catalyseur Al testé en isomérisation selon les conditions de l'exemple 2 sont reportées dans le tableau 3. La teneur en fluor est de 0,80% en poids. La teneur en platine est de 0,06% en poids.

EXEMPLE 7 : Catalyseur A2 conforme à l'invention.

Le catalyseur A2 diffère du catalyseur Al en ce que, avant extrusion, la zéolithe est défluorée partiellement.

La défluoration est réalisée par deux traitements successifs dans une solution de $NH_4OH$ 0,25 N à 80°C pendant 2 heures. Après chaque traitement le solide est filtré et abondamment lavé à l'eau distillée. Après le dernier lavage il est séché à l'étuve à 120°C. Sa teneur en fluor est alors de 0,2% en poids. La teneur en platine est ajustée de façon à obtenir la même teneur que pour les autres catalyseurs à savoir 0,06% poids. Après le dépôt de platine le catalyseur est calciné sous air et réduit dans les mêmes conditions que celles de l'exemple 2. Le catalyseur ainsi obtenu est référencé A2, ses performances catalytiques sur un test d'isomérisation réalisé selon les conditions de l'exemple 2 sont reportées dans le tableau 3.

EXEMPLE 8 : Catalyseur D de comparaison.

On synthétise en milieu conventionnel une zéolithe de structure MFI de rapport Si/Al = 22 d'après le brevet US 3.702.886. Cette zéolithe ne contient pas de fluor. 0,06% poids de platine sont déposés sur ce support mis en forme comme dans l'exemple 2, par échange cationique. Le catalyseur D est activé et testé dans les mêmes conditions que celles énoncées précédemment (exemple 2), ses performances catalytiques sont reportées dans le tableau 3.

Il apparait clairement que le catalyseur D a des rendements en isomérisation et en C8 aromatiques inférieurs à ceux de catalyseurs de même rapport Si/Al mais synthétisés en milieu fluorure : catalyseurs Al et A2.

EXEMPLE 9 : Catalyseur F de comparaison.

On synthétise en milieu conventionnel une zéolithe de structure MFI de rapport Si/Al = 240 en utilisant la procédure décrite dans le brevet US 3.702.886. Cette zéolithe subit une calcination à 550°C suivie de trois échanges en milieu $NH_4NO_3$ 3 N. Le solide est ensuite soumis à un traitement à 450°C sous une atmosphère contenant $CHF_3$ pendant 4 heures. La teneur en fluor atteinte à l'issue de ce traitement est de 0,15% en poids. Ce solide est ensuite mis en forme, puis soumis à un échange cationique, afin de déposer 0,06% poids de platine, à une calcination et une réduction dans les mêmes conditions que celles décrites dans l'exemple 2. Les performances catalytiques du catalyseur F ainsi préparé, testé en isomérisation selon les conditions de l'exemple 2, sont reportées dans le tableau 3. Il apparait que ce catalyseur F présente des performances catalytiques inférieures à celle du catalyseur B2 selon l'invention dont le rapport Si/Al et la teneur en fluor sont voisins de ceux du catalyseur F.

EXEMPLE 10 : Catalyseur B4 conforme à l'invention.

Le catalyseur B4 diffère du catalyseur B1 décrit dans l'exemple 2, en ce que le platine est déposé non plus sur la zéolithe mais sur l'alumine par échange anionique avec de l'acide hexachloroplatinique. La teneur en platine sur le catalyseur est de 0,06%. La teneur en fluor est de 0,5%. Le dépôt de platine est comme précédemment suivi d'une calcination sous air à 500°C pendant 2 heures et d'une réduction in situ à 450°C, pendant 2 heures. Les performances de ce catalyseur référencé B4, après 10 heures de fonctionnement figurent dans le tableau 3. Elles sont parfaitement comparables aux performances du catalyseur B1. Les performances de ce catalyseur après 100 heures de fonctionnement figurent dans le tableau 4.

EXEMPLE 11 : Catalyseur B5 conforme à l'invention.

Le catalyseur B5 diffère du catalyseur B4 décrit dans l'exemple 10, en ce qu'il contient 0,3% de platine. Le mode de dépôt est toujours l'échange anionique afin de déposer la totalité du platine sur l'alumine. Ce catalyseur référencé B5 subit les mêmes traitements thermiques que ceux décrits dans l'exemple 10. Ses performances après 10 heures de fonctionnement figurent dans le tableau 3, et après 100 heures dans le tableau 4. Contrairement au catalyseur B4 contenant peu de platine, les performances du catalyseur B5 sont beaucoup plus stables dans le temps.

EXEMPLE 12 : Catalyseur B0 non conforme à l'invention.

Le catalyseur B0, diffère de tous les catalyseurs décrits précédemment en ce qu'il ne contient pas du tout de platine. Il est testé en isomérisation du m-xylène directement après mise en forme. Ses performances après 100 heures de marche figurent dans le tableau 4. Elles ne sont pas mesurables ; le catalyseur est totalement désactivé.

EXEMPLE 13 : Catalyseur B6, conforme à l'invention.

Le catalyseur B6, diffère du catalyseur B1, décrit dans l'exemple 2 en ce que le métal du groupe VIII déposé n'est plus le platine mais le palladium. 0,06% poids de palladium sont déposés à partir de chlorure de palladium, en présence d'acide chlorhydrique comme agent compétiteur. Le catalyseur ainsi obtenu est ensuite soumis à une calcination sous air à 500°C pendant 2 heures suivie d'une réduction in situ à 450°C pendant 2 heures. Ses performances en isomérisation du m-xylène figurent dans le tableau 3. Elles sont parfaitement comparables à celles du catalyseur B1.

| Catalyseur | B1 | B2 | B3 | C | A1 | A2 | D | F | B4 | B5 | B6 |
|---|---|---|---|---|---|---|---|---|---|---|---|
| Si/Al atomique | 240 | 240 | 240 | 240 | 22 | 22 | 22 | 220 | 240 | 240 | 240 |
| % F | 0,5 | 0,1 | 0,02 | 0 | 0,8 | 0,2 | 0 | 0,15 | 0,5 | 0,5 | 0,5 |
| Conversion du m-xylène (%) | 48 | 51,4 | 47,2 | 48,2 | 53,4 | 40,7 | 42 | 50,0 | 48,2 | 48 | 47,2 |
| Sélectivité Isomérisation (%) | 94,0 | 83,2 | 81,1 | 81,3 | 74,7 | 71,0 | 65,3 | 82 | 94,3 | 95,8 | 96,4 |
| Approche à l'équilibre sur le p-xylène | 100 | 100 | 100 | 100 | 100 | 87,6 | 75,6 | 100 | 100 | 100 | 100 |
| Rendement isomérisation (%) | 45,1 | 42,7 | 38,3 | 39,2 | 40,0 | 29,0 | 27,4 | 41 | 45,4 | 46,0 | 45,5 |
| Rendement $C_8$ aromatique (%) | 98,0 | 92,0 | 88,0 | 88,5 | 92,2 | 91,6 | 85,1 | 90,0 | 98,0 | 98,0 | 98,0 |

TABLEAU 3 : Comparaison des différents catalyseurs après 10 heures de fonctionnement

(T°C = 400 ; p (b) = 15 ; $H_2$/HC = 4 ; pph = 3,6)

| Catalyseur | B0 | B4 | B5 |
|---|---|---|---|
| Si/Al atomique | 240 | 240 | 240 |
| % F | 0,5 | 0,5 | 0,5 |
| % Pt | 0 | 0,06 | 0,3 |
| Conversion du m-xylène (%) | 0 | 45 | 48 |
| Sélectivité isomérisation (%) | 0 | 92,8 | 95,8 |
| Approche à l'équilibre sur le p-xylène | 0 | 100 | 100 |
| Rendement isomérisation (%) | 0 | 41,8 | 46,0 |
| Rendement C8 aromatique (%) | 0 | 96,5 | 98,0 |

TABLEAU 4 : Comparaison des différents catalyseurs après 100 heures de fonctionnement

## Revendications

1. Catalyseur du type aluminosilicate caractérisé par la composition suivante exprimée en poids :
a) 0,01 à 1,5% d'au moins un métal choisi parmi le platine et le palladium,
b) 0 à 99,49% d'une matrice choisie dans le groupe formé par l'alumine, la silice, la magnésie, une argile et toute combinaison d'au moins deux des composés précités et,
c) 0,5 à 99,99% d'une zéolithe ayant un rapport molaire $SiO_2/Al_2O_3$ compris entre 12 et 1000,
ladite zéolithe ayant été synthétisée en milieu fluorure et ayant une teneur en fluor comprise entre 0,02 et 1,5% en poids, la dite zéolithe ayant un diagramme de diffraction des rayons X présenté dans le tableau ci-après :

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08 - 11,26 | FF | 4,06 - 4,10 | ff | 2,772 - 2,793 | ff |
| 9,94 - 10,20 | mf | 3,99 - 4,05 | f | 2,725 - 2,749 | ff |
| 9,68 - 9,90 | f | 3,83 - 3,89 | F | 2,677 - 2,697 | ff |
| 8,98 - 9,08 | ff | 3,80 - 3,86 | m | 2,648 - 2,670 | ff |
| 8,00 - 8,09 | ff | 3,74 - 3,78 | mf | 2,605 - 2,619 | ff |
| 7,40 - 7,52 | ff | 3,70 - 3,74 | mf | 2,581 - 2,597 | ff |
| 7,03 - 7,22 | ff | 3,63 - 3,67 | mf | 2,545 - 2,557 | ff |
| 6,64 - 6,84 | f | 3,58 - 3,62 | ff | 2,508 - 2,526 | ff |
| 6,30 - 6,42 | f | 3,46 - 3,50 | ff | 2,479 - 2,501 | ff |
| 5,95 - 6,07 | f | 3,42 - 3,46 | f | 2,407 - 2,419 | ff |
| 5,67 - 5,79 | f | 3,38 - 3,42 | ff | 2,393 - 2,401 | ff |
| 5,54 - 5,61 | f | 3,33 - 3,37 | f | 2,326 - 2,340 | ff |
| 5,32 - 5,42 | ff | 3,29 - 3,33 | ff | 2,314 - 2,332 | ff |
| 5,10 - 5,23 | ff | 3,23 - 3,27 | ff | 2,195 - 2,209 | ff |
| 5,01 - 5,08 | f | 3,16 - 3,20 | ff | 2,104 - 2,120 | ff |
| 4,95 - 5,03 | f | 3,12 - 3,16 | ff | 2,077 - 2,095 | ff |
| 4,84 - 4,93 | ff | 3,08 - 3,12 | ff | 2,070 - 2,084 | ff |
| 4,59 - 4,64 | ff | 3,03 - 3,07 | f | 2,004 - 2,022 | f |
| 4,44 - 4,50 | ff | 2,976 - 3,020 | f | 1,985 - 2,005 | f |
| 4,34 - 4,40 | f | 2,943 - 2,962 | f | 1,944 - 1,964 | ff |
| 4,23 - 4,29 | f | 2,855 - 2,881 | ff | 1,907 - 1,922 | ff |
| | | | | 1,866 - 1,881 | ff |

FF = très fort ; F = fort ; mF = moyen à fort ; m = moyen
mf = moyen à faible ; f = faible ; ff = très faible

2. Catalyseur selon la revendication 1 dans lequel la teneur en platine est comprise entre 0,03 et 0,4% en poids.

3. Catalyseur selon l'une des revendications 1 à 2 dans lequel la teneur en zéolithe est comprise entre 40 et 90% en poids.

4. Catalyseur selon l'une des revendications 1 à 3 dans lequel la teneur en matrice est comprise entre 10 et 60% en poids.

5. Catalyseur selon l'une des revendications 1 à 4 lequel la teneur en fluor par rapport à ladite zéolithe est comprise entre 0,2 et 1% en poids.

6. Utilisation du catalyseur selon l'une des revendications 1 à 5 dans la réaction d'isomérisation d'un hydrocarbure C8 aromatique.

## Patentansprüche

1. Katalysator vom Typ Aluminosilikat, **gekennzeichnet** durch die folgende Zusammensetzung, ausgedrückt in Gewicht :

a) 0,01 bis 1,5% wenigstens eines Metalls, das aus Platin und Palladium gewählt ist,

b) 0 bis 99,49% einer Matrix, die gewählt ist aus der Gruppe gebildet aus Aluminiumoxyd, Siliciumoxyd, Magnesiumoxyd, einem Ton und jeder beliebigen Kombination wenigstens zweier der vorgenannten Verbindungen und

c) 0,5 bis 99,99% eines Zeolith mit einem Molverhältnis $SiO_2/Al_2O_3$ zwischen 12 und 1000,

wobei der Zeolith in Fluoridumgebung synthetisiert worden ist und einen Fluorgehalt Zwischen 0,02 und 1,5 Gew.-% hat und der Zeolith ein Röntgenstrahlen-Diffraktionsdiagram gemäß der nachstehenden Tabelle hat :

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08 - 11,26 | FF | 4,06 - 4,10 | ff | 2,772 - 2,793 | ff |
| 9,94 - 10,20 | mf | 3,99 - 4,05 | f | 2,725 - 2,749 | ff |
| 9,68 - 9,90 | f | 3,83 - 3,89 | F | 2,677 - 2,697 | ff |
| 8,98 - 9,08 | ff | 3,80 - 3,86 | m | 2,648 - 2,670 | ff |
| 8,00 - 8,09 | ff | 3,74 - 3,78 | mf | 2,605 - 2,619 | ff |
| 7,40 - 7,52 | ff | 3,70 - 3,74 | mf | 2,581 - 2,597 | ff |
| 7,03 - 7,22 | ff | 3,63 - 3,67 | mf | 2,545 - 2,557 | ff |
| 6,64 - 6,84 | f | 3,58 - 3,62 | ff | 2,508 - 2,526 | ff |
| 6,30 - 6,42 | f | 3,46 - 3,50 | ff | 2,479 - 2,501 | ff |
| 5,95 - 6,07 | f | 3,42 - 3,46 | f | 2,407 - 2,419 | ff |
| 5,67 - 5,79 | f | 3,38 - 3,42 | ff | 2,393 - 2,401 | ff |
| 5,54 - 5,61 | f | 3,33 - 3,37 | f | 2,326 - 2,340 | ff |
| 5,32 - 5,42 | ff | 3,29 - 3,33 | ff | 2,314 - 2,332 | ff |
| 5,10 - 5,23 | ff | 3,23 - 3,27 | ff | 2,196 - 2,209 | ff |
| 5,01 - 5,08 | f | 3,16 - 3,20 | ff | 2,104 - 2,120 | ff |
| 4,95 - 5,03 | f | 3,12 - 3,16 | ff | 2,077 - 2,095 | ff |
| 4,84 - 4,93 | ff | 3,08 - 3,12 | ff | 2,070 - 2,084 | ff |
| 4,59 - 4,64 | ff | 3,03 - 3,07 | f | 2,004 - 2,022 | f |
| 4,44 - 4,50 | ff | 2,976 - 3,020 | f | 1,985 - 2,005 | f |
| 4,34 - 4,40 | f | 2,943 - 2,962 | f | 1,944 - 1,964 | ff |
| 4,23 - 4,29 | f | 2,856 - 2,881 | ff | 1,907 - 1,922 | ff |
| | | | | 1,866 - 1,881 | ff |

FF = sehr stark; F = stark; mF = mittel bis stark;
m = mittel; mf = mittel bis schwach; f = schwach;
ff = sehr schwach

2. Katalysator nach Anspruch 1, bei dem der Gehalt an Platin zwischen 0,03 und 0,4 Gew.-% beträgt.

3. Katalysator nach einem der Ansprüche 1 bis 2, bei dem der Gehalt an Zeolith zwischen 40 und 90 Gew.-% beträgt.

4. Katalysator nach einem der Ansprüche 1 bis 3, bei dem der Gehalt an Matrix 10 bis 60 Gew.-% beträgt.

5. Katalysator nach einem der Ansprüche 1 bis 4, wobei der Gehalt an Fluor bezogen auf den Zeolithen zwischen 0,2 und 1 Gew.-% beträgt.

6. Verwendung des Katalysators nach einem der Ansprüche 1 bis 5 in der Isomerisierungsreaktion eines aromatischen C8-Kohlenwasserstoffs.

## Claims

1. A catalyst of aluminosilicate type, characterized by the following composition, expressed by weight :

a) 0.01 to 1.5% of at least one metal selected from platinum and palladium,

b) 0 to 99.49% of a matrix selected from the group formed of alumina, silica, megnesia, a clay or any combination of at least two of the above-mentioned compounds, and

c) 0.5 to 99.9% of a zeolite having a $SiO_2/Al_2O_3$ molar ratio from 12 to 1000,

said zeolite, previously synthesized in fluoride medium, having a fluorine content from 0.02 to 1.5% by weight and a X-ray diffraction diagram as shown in the table below :

2. A catalyst according to claim 1, wherein the platinum content ranges from 0.03 to 0.4%, by weight.

3. A catalyst according to one of claims 1 and 2, wherein the zeolite content ranges from 40 to 90% by weight.

4. A catalyst according to one of claims 1 to 3, wherein the matrix content ranges from about 10 to 60% by weight.

5. A catalyst according to one of claims 1 to 4, wherein the fluorine content in proportion to said zeolite ranges from 0.2 to 1% by weight.

6. The use of the catalyst according to one of claims 1 to 5, for isomerizing a $C_8$ aromatic hydrocarbon.

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08 - 11,26 | FF | 4,06 - 4,10 | ff | 2,772 - 2,793 | ff |
| 9,94 - 10,20 | mf | 3,99 - 4,05 | f | 2,725 - 2,749 | ff |
| 9,68 - 9,90 | f | 3,83 - 3,89 | F | 2,677 - 2,697 | ff |
| 8,98 - 9,08 | ff | 3,80 - 3,86 | m | 2,648 - 2,670 | ff |
| 8,00 - 8,09 | ff | 3,74 - 3,78 | mf | 2,605 - 2,619 | ff |
| 7,40 - 7,52 | ff | 3,70 - 3,74 | mf | 2,581 - 2,597 | ff |
| 7,03 - 7,22 | ff | 3,63 - 3,67 | mf | 2,545 - 2,557 | ff |
| 6,64 - 6,84 | f | 3,58 - 3,62 | ff | 2,508 - 2,526 | ff |
| 6,30 - 6,42 | f | 3,46 - 3,50 | ff | 2,479 - 2,501 | ff |
| 5,95 - 6,07 | f | 3,42 - 3,46 | f | 2,407 - 2,419 | ff |
| 5,67 - 5,79 | f | 3,38 - 3,42 | ff | 2,393 - 2,401 | ff |
| 5,54 - 5,61 | f | 3,33 - 3,37 | f | 2,326 - 2,340 | ff |
| 5,32 - 5,42 | ff | 3,29 - 3,33 | ff | 2,314 - 2,332 | ff |
| 5,10 - 5,23 | ff | 3,23 - 3,27 | ff | 2,195 - 2,209 | ff |
| 5,01 - 5,08 | f | 3,16 - 3,20 | ff | 2,104 - 2,120 | ff |
| 4,95 - 5,03 | f | 3,12 - 3,16 | ff | 2,077 - 2,095 | ff |
| 4,84 - 4,93 | ff | 3,08 - 3,12 | ff | 2,070 - 2,084 | ff |
| 4,59 - 4,64 | ff | 3,03 - 3,07 | f | 2,004 - 2,022 | f |
| 4,44 - 4,50 | ff | 2,976 - 3,020 | f | 1,985 - 2,005 | f |
| 4,34 - 4,40 | f | 2,943 - 2,962 | f | 1,944 - 1,964 | ff |
| 4,23 - 4,29 | f | 2,855 - 2,881 | ff | 1,907 - 1,922 | ff |
| | | | | 1,866 - 1,881 | ff |

FF = very strong ; F = strong ; mF = middle to strong ; m = middle
mf = middle to low ; f = low ; ff = very low.

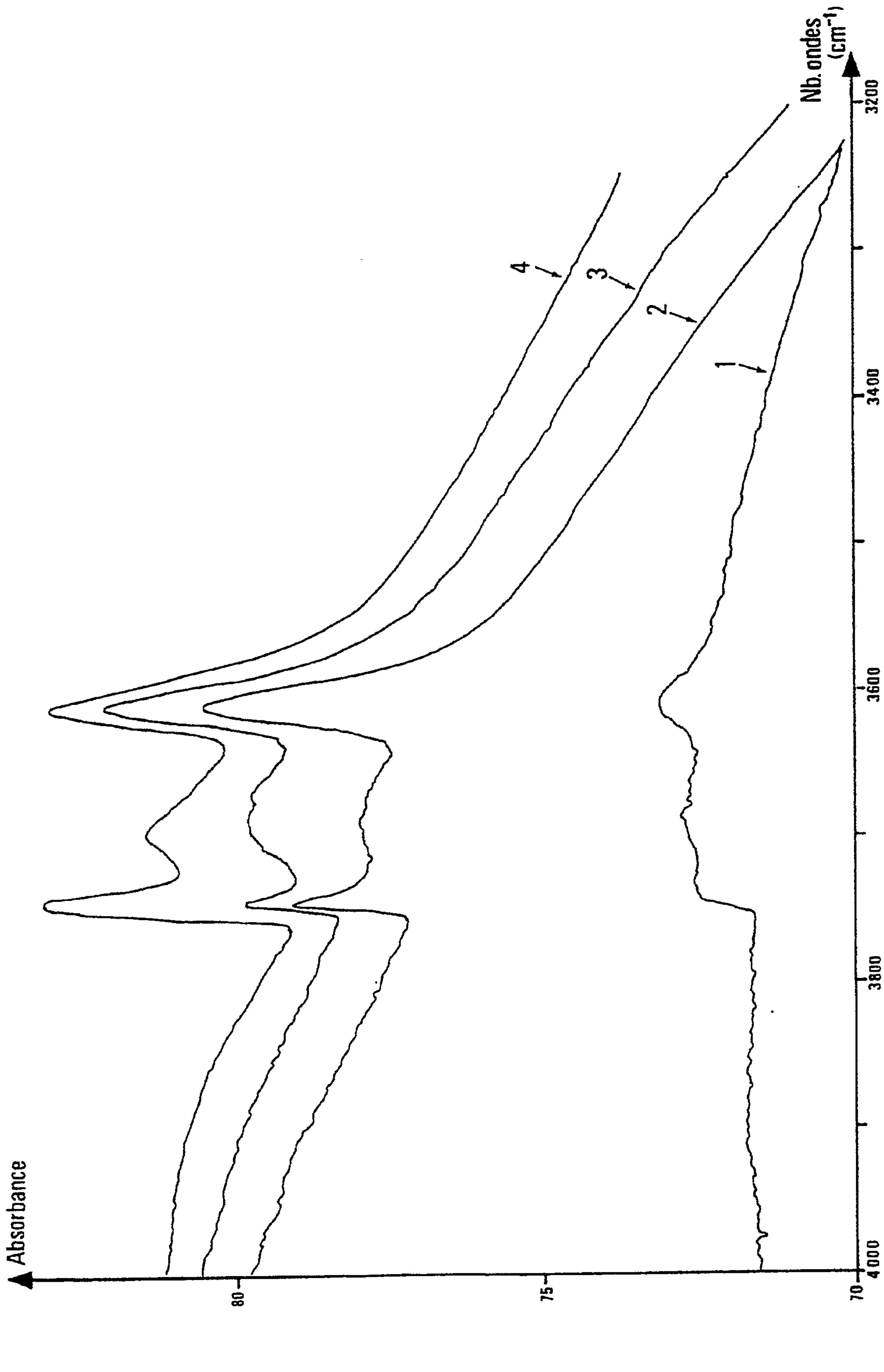
Absorbance
Nb. ondes (cm-1)
80
75
70
4000
3800
3600
3400
3200
1
2
3
4